# EUROPEAN PATENT APPLICATION

(11) **EP 1 849 771 A1**
(43) Date of publication of application: **31.10.2007**
(21) Application number: 06384008.6
(22) Date of filing: 28.04.2006
(51) Int. Cl.: C07D 209/52, A61K 31/403, A61P 25/28

(54) **Bicyclic tetrahydropyrrole compounds**

(71) Applicant: LABORATORIOS DEL DR. ESTEVE, S.A., 08041 Barcelona (ES)
(72) Inventor: Pericas Brondo, Miguel Angel Institut Català d'Investigació Quimica(ICIQ), 43007 Tarragona (ES); Torrens-Jover, Antonio, 08221 Tarressa (ES)
(74) Representative: Peters, Hajo

(57) **Abstract**

The present invention relates to substituted bicyclic tetrahydropyrrole compounds of general formula (I), 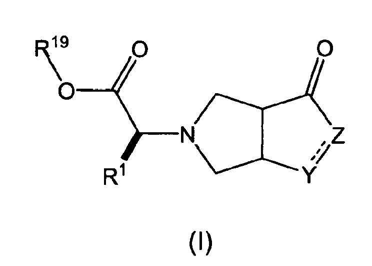 methods for their preparation, medicaments comprising these compounds as well their use in the manufacture of a medicament for the treatment of humans and animals.

## Description

### Field of the invention

The present invention relates to compounds having pharmacological activity towards the sigma (σ) receptor, and more particularly to some bicyclic tetrahydropyrrole derivatives, to processes of preparation of such compounds, to medicaments comprising them, and to their use in therapy and prophylaxis, in particular for the treatment of Alzheimer's disease.

### Background of the invention

Alzheimer's disease is a progressive, irreversible brain disorder with no known cause or cure. Symptoms of the disease include memory loss, confusion, impaired judgment, personality changes, disorientation, and loss of language skills. Always fatal, Alzheimer's disease is the most common form of irreversible dementia.

According to the American Health Assistance Foundation (AHAF), more than 4.5 million Americans are believed to have Alzheimer's disease and by 2050, the number could increase to 13.2 million. In every nation where life expectancy has increased, so has the incidence of Alzheimer's disease. Alzheimer's disease is becoming tragically common. It is estimated that there are currently 18 million people worldwide with Alzheimer's disease. This figure is projected to nearly double by 2025 to 34 million people.

Considering the fact that there is at present no effective treatment for this fatal disease, it is an imperative to find new solutions to treat AD.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

Therefore, compounds binding to the sigma receptor and which are suitable for modulating these receptors are useful in the prevention and/or the treatment of diseases associated with the sigma receptor.

Recently it has been found that the sigma-1 receptor may be involved in the pathologenesis of Alzheimer's disease (Uchida et al., Am J Geriatr Psychiatry 2005; 13:1062-1066).

Thus, it was an objective of the present invention to provide new compounds for the use as active ingredients in medicaments. In particular, these active ingredients should be suitable to modulate the sigma receptor, more particularly the sigma-1 receptor.

Said objective was achieved by providing substituted bicyclic tetrahydropyrrolidine compounds of general formula (I) given below, their stereoisomers, corresponding salts and corresponding solvates thereof.

Thus, one of the aspect of the present inventon relates to substituted bicyclic tetrahydropyrrolidine compounds of general formula (I) 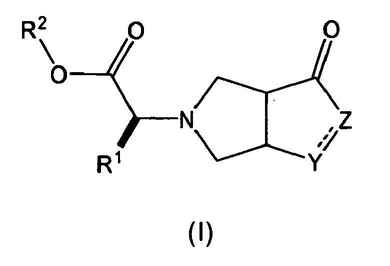 wherein
R¹ represents a hydrogen atom; an unbranched or branched, saturated or unsaturated, optionally at least mono-substituted aliphatic radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; an optionally, at least mono-substituted benzhydryl group;
wherein the bond between Y and Z may be unsaturated (Y=Z) or saturated (Y-Z);
in case of Y and Z being (Y=Z), Y represents CH and Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
in case of Y and Z being (Y-Z), Y represents CH₂; C-R¹¹R¹²; a CH-(C=O)-R¹⁶ group; a CH-(SO₂)-R¹⁷ group; CH-(SO₂)-NR¹⁸R^{18a} group; or a CH-(C=O)-NR¹⁸R^{18a} group and Z represents CH-R⁶; CH-CHR⁷R^{7a}; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group;
R², R³, R⁴, and R⁶ represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁵, R^{5a}, identical or different, represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁷, R^{7a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁸, R⁹, R¹³, R¹⁴, R¹⁵, R¹⁶ and R¹⁷ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁰, R^{10a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹¹ and R¹², identical or different, represent represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; a -(SO₂)-R¹³ -group; or a -NR ¹⁴R¹⁵ -group;
R¹⁸ and R^{18a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁹ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a preferred aspect of the invention the following proviso applies:
if R¹⁹ is methyl,
and Y and Z are (Y=Z)
and Y represent CH,

Z may not represent a CH group.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The term "condensed" according to the present invention means that a ring or ring-system is attached to another ring or ring-system, whereby the terms "annulated" or "annelated" are also used by those skilled in the art to designate this kind of attachment.

The term "ring system" according to the present invention refers to ring sytems comprises saturated, unsaturated or aromatic carbocyclic ring sytems which contain optionally at least one heteroatom as ring member and which are optionally at least mono-substituted. Said ring systems may be condensed to other carbocyclic ring systems such as aryl groups, naphtyl groups, heteroaryl groups, cycloalkyl groups, etc.

Cyclyl groups/radicals, as defined in the present invention, comprise any saturated, unsaturated or aromatic carbocyclic ring sytems which contain optionally at least one heteroatom as ring member and which are optionally at least mono-substituted. Cyclyl groups preferably comprise aryl, heteroaryl, cyclyl, heterocylcyl and/or spiro ring systems.

Heterocyclyl groups/radicals, as defined in the present invention, comprise any saturated, unsaturated or aromatic carbocyclic ring sytems which are optionally at least mono-substituted and which contain at least one heteroatom as ring member. Preferred heteroatoms for these heterocyclyl groups are N, S or O.

Aliphatic radicals/groups, as referred to in the present invention, are optionally mono-or polysubstituted and may be branched or unbranched, saturated or unsaturated. Unsaturated aliphatic groups, as defined in the present invention, include alkyl, alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl. Preferred substituents for aliphatic radicals, according to the present invention, are a C₁₋₄ alkyl group, a linear or branched C₁₋₆ alkoxy group, F, Cl, l, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group.

Alkyl radicals, as referred to in the present invention, are saturated aliphatic radicals. They may be linear or branched and are optionally substituted.

Cycloalkyl radicals, as referred to in the present invention, are understood as meaning saturated and unsaturated (but not aromatic), cyclic hydrocarbons, which can optionally be unsubstituted, mono- or polysubstituted. In these radicals, for example C₃₋₄-cycloalkyl represents C₃- or C₄-cycloalkyl, C₃₋₅-cycloalkyl represents C₃-, C₄- or C₅-cycloalkyl, etc. With respect to cycloalkyl, the term also includes saturated cycloalkyls in which optionally at least one carbon atom may be replaced by a heteroatom, preferably S, N, P or O. However, mono- or polyunsaturated, preferably monounsaturated, cycloalkyls without a heteroatom in the ring also in particular fall under the term cycloalkyl as long as the cycloalkyl is not an aromatic system.

Examples for cycloalkyl radicals preferably include but are not restricted to cyclopropyl, 2-methylcyclopropyl, cyclopropylmethyl, cyclobutyl, cyclopentyl, cyclopentylmethyl, cyclohexyl, cycloheptyl, cyclooctyl, acetyl, tert-butyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine.

Cycloalkyl radicals, as defined in the present invention, are optionally mono-or polysubstituted by substitutents independently selected from a C₁₋₄ alkyl group, a linear or branched C₁₋₆ alkoxy group, F, Cl, I, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group.

An aryl radical, as referred to in the present invention, is understood as meaning ring systems with at least one aromatic ring but without heteroatoms even in only one of the rings. These aryl radicals may optionally be mono-or polysubstituted by substitutents independently selected from a C₁₋₄ alkyl group, a linear or branched C₁₋₆ alkoxy group, an optionally at least mono-substituted phenyl group, F, Cl, l, Br, CF₃, CH₂F, CHF₂, CN, OH, SH, NH₂, oxo, (C=O)R', SR', SOR', SO₂R', N(C=O)-OR', NHR', NR'R" whereby R' and optionally R" for each substitutent independently represents a linear or branched C₁₋₆-alkyl group. Preferred examples of aryl radicals include but are not restricted to phenyl, naphthyl, fluoranthenyl, fluorenyl, tetralinyl or indanyl or anthracenyl radicals, which may optionally be mono- or polysubstituted, if not defined otherwise.

An alkyl-aryl radical, as defined in the present invention, comprises a linear or branched, optionally at least mono-substituted alkyl chain which is bonded to an aryl group, as defined above. A preferred alkyl-aryl radical is a benzyl group, wherein the alkyl chain is optionally branched or substituted.

A heteroaryl radical is understood as meaning heterocyclic ring systems which have at least one aromatic ring and may optionally contain one or more heteroatoms from the group consisting of nitrogen, oxygen and/or sulfur and may optionally be mono-or polysubstituted by substitutents independently selected from a C₁₋₄ alkyl group, a linear or branched C₁₋₆ alkoxy group, F, Cl, l, Br, CF₃, CH₂F, CHF₂, CN, OH, SH, NH₂, oxo, (C=O)R', SR', SOR', SO₂R', NHR', NR'R" whereby R' and optionally R" for each substitutent independently represents a linear or branched C₁₋₆-alkyl group. Preferred examples of heteroaryls include but are not restricted to furan, benzofuran, thiophene, benzothiophene, pyrrole, pyridine, pyrimidine, pyridazine, pyrazine, quinoline, isoquinoline, phthalazine, benzo-1,2,5-thiadiazole, benzothiazole, indole, benzotriazole, benzodioxolane, benzodioxane, benzimidzole, carbazole and quinazoline.

With respect to compounds of general formula (I) of the present invention, Y and Z may form an unsaturated (Y=Z) or a saturated (Y-Z) bond which is illustrated below.

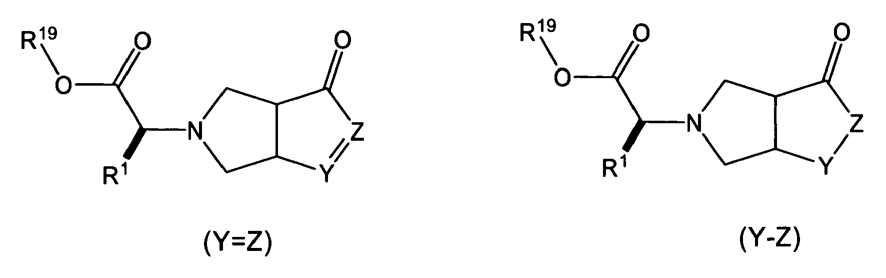

A preferred embodiment of the present invention are compounds of general formula (1a) 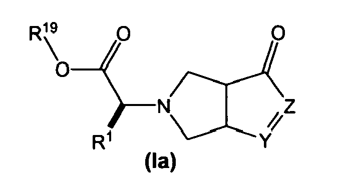 in which
Y represents CH and Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
and
R¹, R⁶, R⁷, R^{7a}, R⁸, R⁹ R¹⁰, R^{10a} and R¹⁹ are defined as above.

Another preferred embodiment of the present invention are compounds of general formula (lb), 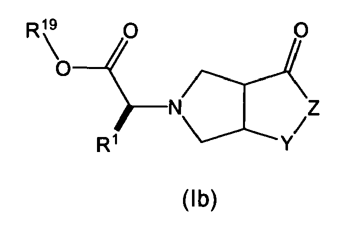 in which
Y represents CH₂; C-R¹¹R¹²; a CH-(C=O)-R ¹⁶ group; a CH-(SO₂)-R¹⁷ group; CH-(S0₂)-NR¹⁸R^{18a} group; or a CH-(C=O)-NR¹⁸R^{18a} group and Z represents CH-R⁶; CH-CHR⁷R^{7a}; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group;
and
R¹, R⁶, R⁷, R^{7a}, R⁸, R⁹ R¹⁰, R^{10a}, R¹¹, R¹², _{R}¹⁶, R¹⁷ _{R}¹⁸, _{R}^{18a} and _{R}¹⁹, are defined as above.

A preferred embodiment of the present invention are compounds of general formula (Ia), wherein
Z represents C-R⁶, thus leading to the compounds being of a structure according to general formula (Iaa) 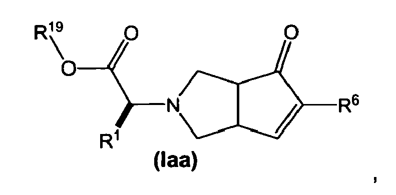 with R¹, R⁶ and R¹⁹ being defined as above.

Another preferred embodiment of the present invention are compounds of general formula (I), (Ia), (Ib) and (Iaa) wherein
R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R⁶ represents a hydrogen atom; a linear or branched, saturated, optionally at least mono-substituted aliphatic group; an optionally at least mono-substituted aryl group;
most preferably R⁶ represents a linear or branched, saturated, optionally at least mono-substituted C₁₋₆-alkyl group; an optionally at least mono-substituted phenyl group.

Another preferred embodiment of the present invention are compounds of general formula (I), (Ia), (Ib) or (Iaa) wherein
R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R¹ represents a hydrogen atom; a linear or branched, saturated, optionally at least mono-substituted aliphatic group; an optionally at least mono-substituted aryl group;
most preferably R¹ represents a linear or branched, saturated, optionally at least mono-substituted C₁₋₆-alkyl group.

Another preferred embodiment of the present invention are compounds of general formula (I), (Ia), (Ib) and (Iaa) wherein
R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system;
more preferably R¹⁹ represents a hydrogen atom; a linear or branched, saturated, optionally at least mono-substituted aliphatic group; an unbranched or branched, saturated or unsaturated, optionally at least mono-substituted alkoxy radical; an optionally at least mono-substituted aryl group;
most preferably R¹⁹ represents a linear or branched, saturated, optionally at least mono-substituted C₁₋₄-alkyl group; an optionally at least mono-substituted phenyl group.

A highly preferred embodiment of the present invention are compounds of general formula (Iaa) 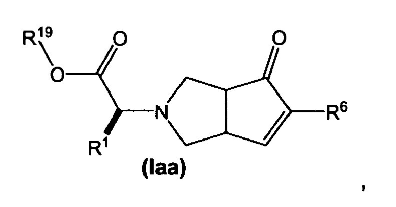 wherein
R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R⁶ represents a hydrogen atom; a linear or branched, saturated, optionally at least mono-substituted aliphatic group; an optionally at least mono-substituted aryl group;
most preferably R⁶ represents a linear or branched, saturated, optionally at least mono-substituted C₁₋₆-alkyl group; an optionally at least mono-substituted phenyl group.
and/or
R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R¹ represents a hydrogen atom; a linear or branched, saturated, optionally at least mono-substituted aliphatic group; an optionally at least mono-substituted aryl group;
most preferably R¹ represents a linear or branched, saturated, optionally at least mono-substituted C₁₋₆-alkyl group.
and/or
R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system;
more preferably R¹⁹ represents a hydrogen atom; a linear or branched, saturated, optionally at least mono-substituted aliphatic group; an unbranched or branched, saturated or unsaturated, optionally at least mono-substituted alkoxy radical; an optionally at least mono-substituted aryl group;
most preferably R¹⁹ represents a linear or branched, saturated, optionally at least mono-substituted C₁₋₄-alkyl group; an optionally at least mono-substituted phenyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Most highly preferred are compounds of general formula (l) as described above, selected from the group consisting of:
[1] (R)-ethyl 2-((3aR.6aS)-4-oxo-5-pheny)-1,3a,4,6a-tetrahydrocyclopenta[c]pyrrol-2(1H)-yl)propanoate
[2] (R)-ethyl 2-((3aS.6aR)-4-oxo-5-phenyl-1,3a,4,6a-tetrahydrocydopenta[c]pyrrol-2(1H)-yl)propanoate,
[3] (S)-methyl 2-((3aR,6aS)-4-oxo-5-phenyl-1,3a,4,6a-tetrahydrocyclopenta[c]pyrrol-2(1H)-yl)-2-phenylacetate,
[4] (S)-methyl 2-((3aS,6aR)-4-oxo-5-phenyl-1,3a,4,6a-tetrahydrocyclopenta[c]pyrrol-2(1H)-yl)-2-phenylacetate,
[5] (S)-methyl 2-((3aR,6aS)-5-butyl-4-oxo-1,3a,4,6a-tetrahydrocyclopenta[c]pyrrol-2(1H)-yl)-2-phenylacetate,
[6] (S)-methyl 2-((3aS,6aR)-5-butyl-4-oxo-1,3a,4,6a-tetrahydrocyclopenta[c]pyrrol-2(1H)-yl)-2-phenylacetate,
[7] (R)-ethyl 2-((3aR,6aS)-5-butyl-4-oxo-1,3a,4,6a-tetrahydrocyclopenta[c]pyrrol-2(1H)-yl)propanoate,
[8] (R)-ethyl 2-((3aS,6aR)-5-butyl-4-oxo-1,3a,4,6a-tetrahydrocyclopenta[c]pyrrol-2(1H)-yl)propanoate,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Another aspect of the present invention refers to a process for obtaining substituted bicyclic tetrahydropyrrole compounds of general formula (1a), characterized in that at least one substituted pyrroline compound of general formula (II), 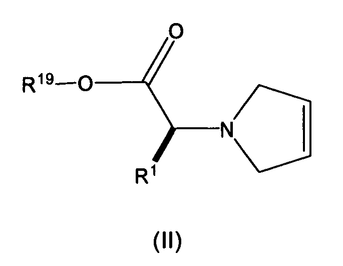 wherein R¹ has the meaning given above, is reacted in presence of a catalyst such as e.g. Co₂(CO)_{8,} an apolar dissolvent such as e.g. DCE (dichloroethane) and an additive such as e.g. DMSO at a reflux temperature between 20 and 100°C, preferably between 50 and 90°C, most preferably between 80 and 90°C, with a compound of general formula (III), 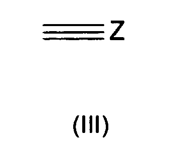 wherein Z represents a CH-R⁶ group; a CH-CHR⁷R^{7a} group; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group, to give compounds of general formula (1a), 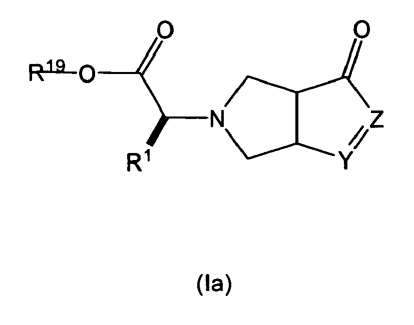 wherein the bond between Y and Z is unsaturated (Y=Z) in which Y represents a CH group and Z has the meaning as defined above.

A general scheme for compounds of general formula (1a) with Y and Z forming an unsaturated bond (Y=Z) is given below in scheme (1):

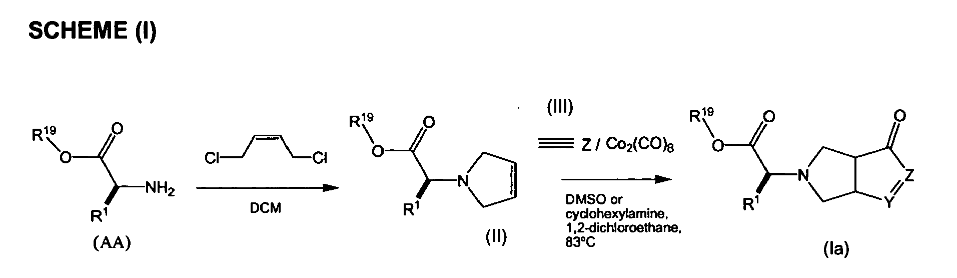

The synthesis of 1-substituted-3-pyrrolines of general formula (II) is performed by treatment of amino acid (ethyl or methyl)esters of general formula (AA) with (Z)-1,4-dichloro-2-butene in an apolar dissolvent such as e.g. dichloromethane (DCM) at a temperature between 10° and 30° Celsius, preferably at room temperature.

Compounds of general formula (I) with Y and Z forming a saturated (Y-Z) bond are obtained by performing a 1,4-addition reaction with a compound of general formula (Ia), 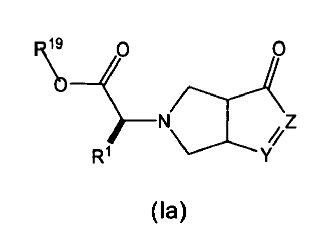 wherein R¹ has the meaning as described above, Z represents a CH-R⁶ group; a CH-CHR⁷R^{7a} group; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group and Y represents a CH group, to give a compound of general formula (Ib), 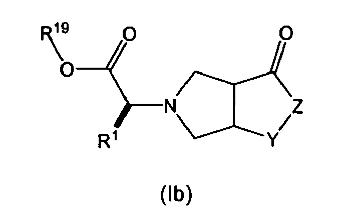 wherein R¹ has the meaning as defined above,Y and Z, as defined above, form a saturated (Y-Z) bond, and Y represents a CH₂ group; a C-R¹¹R¹² group; a CH-(C=O)-R ¹⁶ group; a CH-(SO₂)-R¹⁷ group; CH-(SO₂)-NR¹⁸R^{18a} group; or a CH-(C=O)-NR¹⁸R^{18a} group.

The performance of said 1,4-addition reaction is well known by those skilled in the art and is preferably done in the presence of a catalyst such as Copper iodide and an apolar substrate such as e.g. Et₂O. The reactants in this 1,4-addition may be metallic or non-metallic. Preferably, the reactants are metallic.

Preferred examples of metallic reactants are Y-Li and Y-Mgₓ, wherein Y represents a CH₂ group; a C-R¹¹R¹² group; a CH-(C=O)-R¹⁶ group; a CH-(SO₂)-R¹⁷ group; CH-(SO₂)-NR¹⁸R^{18a} group; or a CH-(C=O)-NR¹⁸R^{18a} group; and x refers to the valency of Mg ,depending on the ligand Y.

A general scheme for compounds of general formula (lb) with Y and Z forming a saturated bond (Y-Z) is given below in scheme (II):

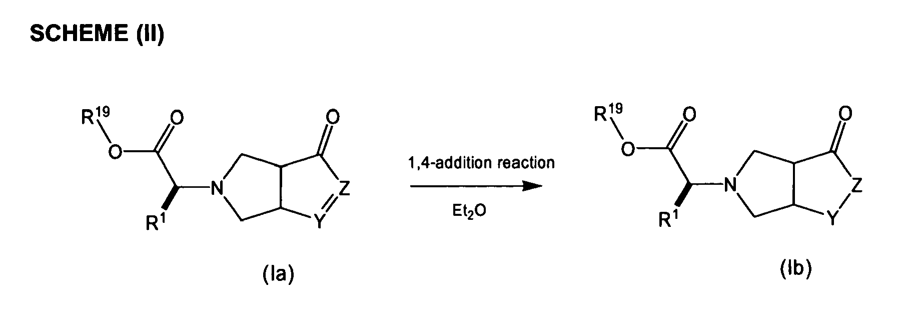

### Synthesis of intermediates:

As described in Scheme I above, the Intermediate (a 1-substituted-3-pyrrolin) of general formula (11) 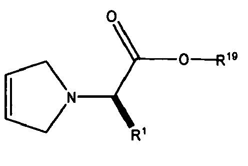 is synthesized by treatment of an amino acid (ethyl or methyl)esters of general formula (AA) according to formula II 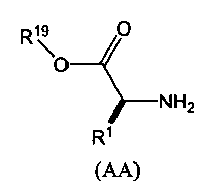 with (Z)-1,4-dichloro-2-butene in an apolar dissolvent such as e.g. dichloromethane (DCM) at a temperature between 10° and 30° Celsius, preferably at room temperature.

During the processes described above the protection of sensitive groups or of reagents may be necessary and/or desirable. The introduction of conventional protective groups as well as their removal may be performed by methods well-known to those skilled in the art.

If the compounds of general formula (I) themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. If there are chiral centers the compounds may be prepared in racemic form, or individual enantiomers may be prepared either by enantiospecific synthesis or by resolution.
Solvates, preferably hydrates, of the compounds of general formula (I), of corresponding stereoisomers, or of corresponding salts thereof may also be obtained by standard procedures known to those skilled in the art.

The purification and isolation of the inventive compounds of general formula (I), of a corresponding stereoisomer, or salt, or solvate or any intermediate thereof may, if required, be carried out by conventional methods known to those skilled in the art, e.g. chromatographic methods or recrystallization.

It has been found that the compounds of general formula (I) and given below, stereoisomers thereof, corresponding salts and corresponding solvates have high affinity to sigma receptors, i.e. they are selective ligands for the sigma receptor and act as modulators, e.g. antagonists, inverse agonists or agonists, on these receptors.

The compounds of general formula (I) given below, their stereoisomers, corresponding salts thereof and corresponding solvates are toxicologically acceptable and are therefore suitable as pharmaceutical active substances for the preparation of medicaments.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the present invention relates to a medicament comprising at least one compound of general formula (I), optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof; or a prodrug thereof.

In an alternative embodiment of the present invention, the medicament comprises at least one compound of general formula (I), said compound being optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Another aspect of the invention is a medicament comprising at least one combination of compounds according to the invention and optionally one or more pharmaceutically acceptable excipients.

In an embodiment according to the invention the medicament is for the prophylaxis and/or treatment of Alzheimer's disease.

In an embodiment according to the invention the medicament is for the prophylaxis and/or treatment of one or more disorders selected from the group consisting of

Said medicament may also comprise any combination of one or more of the compounds of general formula (I) given above, stereoisomers thereof, physiologically acceptable salts thereof or physiologically acceptable solvates thereof.

Another aspect of the present invention is the use of at least one compound of general formula (I) given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, and optionally one or more pharmaceutically acceptable excipients, for the preparation of a medicament for the modulation of sigma receptors, preferably for the prophylaxis and/or treatment of Alzheimer's disease.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice, in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Another aspect of the present invention refers to a method for the prophylaxis and/or treatment of Alzheimer's disease, the method comprising administering to the subject at least one compound of general formula (I) as described above and optionally at least one further active substance and/or optionally at least one auxiliary substance to the subject.

Another aspect of the present invention refers to the use of a compound of general formula (I) in the manufacture of a medicament.

Another aspect of the present invention refers to the use of a compound of general formula (I) in the manufacture of a medicament for the treatment or prophylaxis of a sigma receptor-mediated disease or condition.

Another aspect of the present invention refers to the use mentioned above of a compound of general formula (I) wherein the sigma receptor-mediated disease or condition is Alzheimer's disease.

Another aspect of the present invention refers to the use mentioned above of a compound of general formula (I) wherein the sigma receptor-mediated disease or condition is diarrhoea, lipoprotein disorders, migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety, psychosis or schizophrenia; inflammation, or autoimmune diseases.

Another aspect of the present invention refers to the use mentioned above of a compound of general formula (I) wherein the sigma receptor-mediated disease or condition is a disorder selected from the group consisting of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

Another aspect of the present invention refers to the use mentioned above of a compound of general formula (I) wherein the sigma receptor-mediated disease or condition is a disorder selected from the group consisting of pain, preferably neuropathic pain, inflammatory pain or other pain conditions involving allodynia and/or hyperalgesia.

Another aspect of the present invention refers to the use of a compound of general formula (I) as a pharmacological tool.

The present invention is illustrated below with the aid of examples. These illustrations are given solely by way of example and do not limit the general spirit of the present invention.

### EXAMPLES

**General Procedure for the synthesis of Pauson-Khand adducts.** To a solution of the acetylene (1.1 eq) in 1,2-dichloroethane, was added Co₂⁽CO)₈ (1.1 eq) and the mixture was stirred 2 hours at room temperature. A solution of the pyrroline (1 eq) in 1,2-dichloroethane and the additive (dimethylsulfoxide or cyclohexylamine) (3.5 eq) were added and the mixture was heated at 83°C for 20 hours.

The reaction mixture was filtered through celite and washed with CH₂CI₂. The filtrate was concentrated and the crude was purified by flash chromatography.

The following examples were synthesized according to the general Schemes and descriptions given above and their structure was determined by 1 H-NMR and MS.

### Example 1: (R)-ethyl 2-((3aR,6aS)-4-oxo-5-phenyl-1,3a,4,6a-tetrahydrocyclopenta[c]pyrrol-2(1H)-yl)propanoate

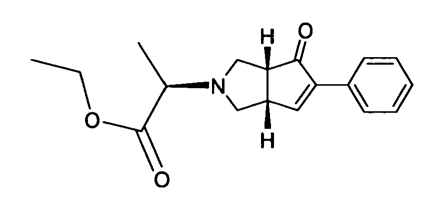

### Example 2: (R)-ethyl 2-((3aS,6aR)-4-oxo-5-phenyl-1,3a,4,6a-tetrahydrocyclopenta[c]pyrrol-2(1H)-yl)propanoate

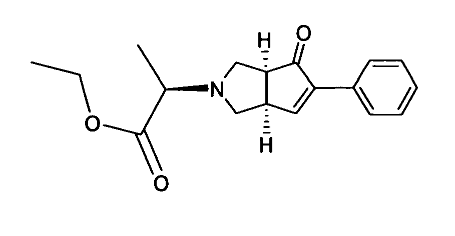

### Example 3: (S)-methyl 2-((3aR,6aS)-4-oxo-5-phenyl-1,3a,4,6a-tetrahydrocyclopenta[c]pyrrol-2(1H)-yl)-2-phenylacetate

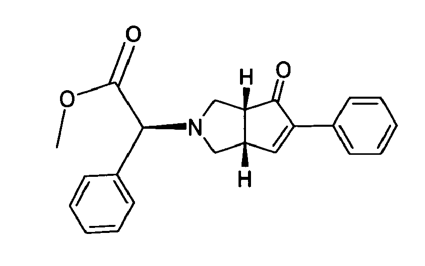

### Example 4: (S)-methyl 2-((3aS,6aR)-4-oxo-5-phenyl-1,3a,4,6a-tetrahydrocyclopenta[c]pyrrol-2(1H)-yl)-2-phenylacetate

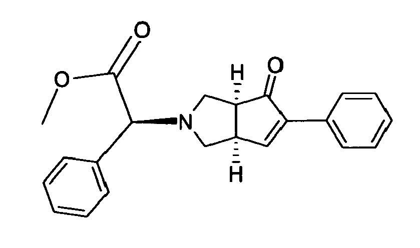

### Example 5: (S)-methyl 2-((3aR,6aS)-5-butyl-4-oxo-1,3a,4,6a-tetrahydrocyclopenta[c]pyrrol-2(1H)-yl)-2-phenylacetate

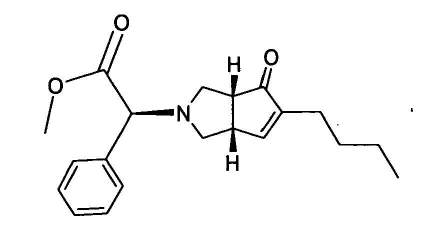

### Example 6: (S)-methyl 2-((3aS,6aR)-5-butyl-4-oxo-1,3a,4,6a-tetrahydrocyclopenta[c]pyrrol-2(1H)-yl)-2-phenylacetate

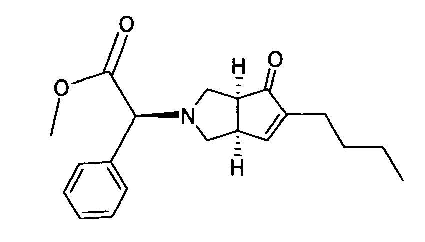

### Example 7: (R)-ethyl 2-((3aR,6aS)-5-butyl-4-oxo-1,3a,4,6a-tetrahydrocyclopenta[c]pyrrol-2(1H)-yl)propanoate

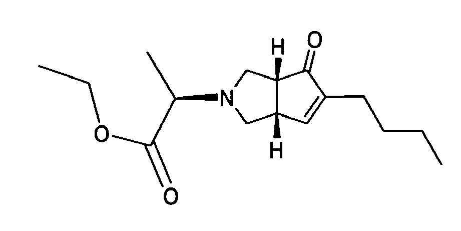

### Example 8: (R)-ethyl 2-((3aS,6aR)-5-butyl-4-oxo-1,3a,4,6a-tetrahydrocyclopenta[c]pyrrol-2(1H)-yl)propanoate

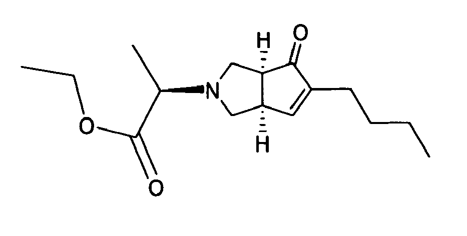

### BIOLOGICAL ACTIVITY

Some representative compounds of the invention are tested for their activity as sigma (sigma-1 and sigma-2) inhibitors. The following protocols are followed:

### Si-qma-1

Brain membrane preparation and binding assays for the σ1-receptor are performed as described (DeHaven-Hudkins et al., 1992) with some modifications. In brief, guinea pig brains are homogenized in 10 vols. (w/v) of Tris-HCl 50 mM 0.32 M sucrose, pH 7.4 is centrifuged at 1000g for 10 min at 4°C and the supernatants collected and centrifuged again at 48000g for 15 min at 4°C. The pellet is resuspended in 10 volumes of Tris-HCI buffer (50 mM, pH 7.4), incubated at 37°C for 30 min, and centrifuged at 48000g for 20 min at 4°C. Following this, the pellet is resuspended in fresh Tris-HCI buffer (50 mM, pH 7.4) and stored on ice until use. Each assay tube contain 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding is defined by addition of a final concentration of 1 µM haloperidol. All tubes are incubated at 37°C for 150 min before termination of the reaction by rapid filtration over Schleicher & Schuell GF 3362 glass fibre filters [previously soaked in a solution of 0,5% polyethylenimine for at least 1 h]. Filters are then washed four times with 4 mL of cold Tris-HCI buffer (50 mM, pH 7.4). Following addition of scintillation cocktail, the samples are allowed to equilibrate overnight. The amount of bound radioactivity is determined by liquid scintillation spectrometry using a Wallac Winspectral 1414 liquid scintillation counter. Protein concentrations are determined by the method of Lowry et al. (1951).

### Sigma-2

Binding studies for σ2-receptor are performed as described (Radesca et al., 1991) with some modifications. In brief, brains from sigma receptor type I (σ1) knockout mice are homogenized in a volume of 10 mUg tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates are then centrifuged at 1000g for 10 min at 4°C, and the supernatants are saved. The pellets are resuspended by vortexing in 2 mUg ice-cold Tris-sucrose buffer and centrifuged again at 1000g for 10 min. The combined 1000g supernatants are centrifuged at 31000g for 15 min at 4°C. The pellets are resuspended by vortexing in 3 mUg 10 mM Tris-HCI, pH 7.4, and the suspension is kept at 25°C for 15 min. Following centrifugation at 31000g for 15 min, the pellets are resuspended by gentle Potter Elvehjem homogenization to a volume of 1.53 mUg in 10 mM Tris-HCI pH 7.4.

The assay tubes contain 10 µL of [³H]-DTG (final concentration of 3 nM), 400 µL of the tissue suspension (5.3 mUg in 50 mM Tris-HCI, pH 8.0) to a final assay volume of 0.5 mL. Non-specific binding is defined by addition of a final concentration of 1 µM haloperidol. All tubes are incubated at 25°C for 120 min before termination of the reaction by rapid filtration over Schleicher & Schuell GF 3362 glass fibre filters [previously soaked in a solution of 0,5% polyethylenimine for at least 1 h]. Filters are washed with three times with 5 mL volumes of cold Tris-HCI buffer (10 mM, pH 8.0). Following addition of scintillation cocktail samples are allowed to equilibrate overnight. The amount of bound radioactivity is determined by liquid scintillation spectrometry using a Wallac Winspectral 1414 liquid scintillation counter. Protein concentrations are determined by the method of Lowry et al. (1951).

### References

DeHaven-Hudkins, D. L., L.C. Fleissner, and F. Y. Ford-Rice, 1992, "Characterization of the binding of [3H](+)pentazocine to σ recognition sites in guinea pig brain", Eur. J. Pharmacol. 227, 371-378.

Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.

Langa, F., Codony X., Tovar V., Lavado A., Giménez E., Cozar P., Cantero M., Dordal A., Hernández E., Pérez R., Monroy X., Zamanillo D., Guitart X., Montoliu LI., 2003, Generation and phenotypic analysis of sigma receptor type l (Sigma1) knockout mice, European Journal of Neuroscience, Vol. 18, 2188-2196.

Lowry, O.H., N.J. Rosebrough, A.L. Farr, and R.J. Randall, 1951, Protein measurement with the Folin phenol reagent, J. Biol. Chem, 193, 265.

Some of the results obtained are shown in table (I).

**Table (I)**

| Example | % Binding σ1 10⁻⁷M | % Binding σ1 10⁻⁸M |
|---|---|---|
| 1 | | |
| 2 | | |
| 3 | | |
| 4 | | |
| 5 | | |
| 6 | | |
| 7 | | |
| 8 | | |

## Claims

1. Substituted bicyclic tetrahydropyrrole compounds of general formula (1) 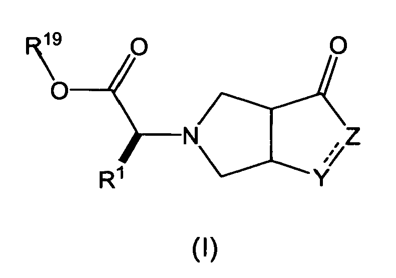 wherein
R¹ represents a hydrogen atom; an unbranched or branched, saturated or unsaturated, optionally at least mono-substituted aliphatic radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; an optionally, at least mono-substituted benzhydryl group;
wherein the bond between Y and Z may be unsaturated (Y=Z) or saturated (Y-Z);
in case of Y and Z being (Y=Z), Y represents CH and Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
in case of Y and Z being (Y-Z), Y represents CH₂; C-R¹¹R¹²; a CH-(C=O)-R¹⁶ group; a CH-(SO₂)-R¹⁷ group; CH-(S0₂)-NR¹⁸R^{18a} group; or a CH-(C=O)-NR¹⁸R^{18a} group and Z represents CH-R⁶; CH-CHR⁷R^{7a}; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group;
R², R³, R⁴, and R⁶ represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R ⁵ , R^{5a}, identical or different, represent a hydrogen atom; a linear or branched, saturated or unsaturated, optionally at least mono-substituted aliphatic group; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁷, R^{7a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R⁸, R⁹, R¹³, R¹⁴ , R¹⁵, R¹⁶ and R¹⁷ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁰, R^{10a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹¹ and R¹², identical or different, represent represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system; a -(SO₂)-R¹³-group; or a -NR¹⁴R¹⁵-group;
R¹⁸ and R^{18a}, identical or different, represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
R¹⁹ represent a hydrogen atom; a linear or branched, saturated or unsaturated, optionally at least mono-substituted aliphatic group; an unbranched or branched, saturated or unsaturated, optionally at least mono-substituted alkoxy radical; a saturated or unsaturated, optionally at least mono-substituted, optionally at least one heteroatom as ring member containing cyclyl group, which may be condensed with an optionally at least mono-substituted mono- or polycyclic ring system; a branched or unbranched alkyl-cycloalkyl group in which either the alkyl group and/or the cycloalkyl group is optionally at least mono-substituted; a branched or unbranched, saturated or unsaturated, optionally at least mono-substituted alkyl-aryl group in which the aryl group may be condensed with another, optionally at least mono-substituted mono- or polycyclic ring system; a branched or unbranched, saturated or unsaturated, optionally at least mono-substituted alkyl-heterocyclyl group in which the heterocyclyl group is optionally condensed with another, at least mono-substituted mono- or polycyclic ring system;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compounds according to claim 1, wherein the compounds are compounds according to formula la, 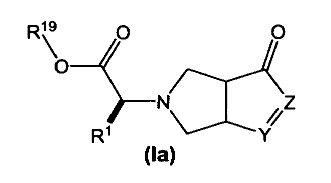 in which
Y represents CH and Z represents C-R⁶; C-CHR⁷R^{7a}; a C-(C=O)-R⁸ group; a C-CH₂(SO₂)-R⁹ group; a C-CH₂(SO₂)-NR¹⁰R^{10a} group; or a C-(C=O)-NR¹⁰R^{10a} group;
and
R¹, R⁶, R⁷, R^{7a}, R⁸, R⁹ R¹⁰, R^{10a} and R¹⁹ are defined as in claim 1.

3. Compounds according to claim 1, wherein the compounds are compounds according to formula lb, 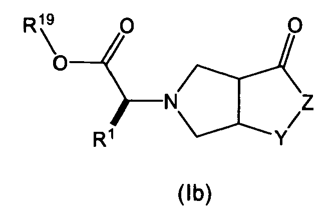 in which
Y represents CH₂; C-R¹¹R¹²; a CH-(C=O)-R¹⁶ group; a CH-(SO₂)-R¹⁷ group; CH-(SO₂)-NR¹⁸R^{18a} group; or a CH-(C=O)-NR¹⁸R^{18a} group and Z represents CH-R⁶; CH-CHR⁷R^{7a}; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group;
and
R¹, R⁶, R⁷, R^{7a}, R⁸, R⁹, R¹⁰, R^{10a}, R¹¹, R¹², R¹⁶, R¹⁷, R¹⁸, R^{18a} and R¹⁹, are defined as in claim 1.

4. Compounds according to one or more of claims 1 or 2, **characterized in that**
Z represents C-R⁶, thus leading to the compounds being of a structure according to general formula (Iaa) 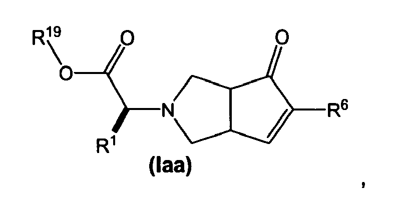 with R¹, R⁶ and R¹⁹ being defined as in claim 1.

5. Compounds according to one or more of claims 1 to 4, **characterized in that**
R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R⁶ represents a hydrogen atom; a linear or branched, saturated, optionally at least mono-substituted aliphatic group; an optionally at least mono-substituted aryl group;
most preferably R⁶ represents a linear or branched, saturated, optionally at least mono-substituted C₁₋₆-alkyl group; an optionally at least mono-substituted phenyl group.

6. Compounds according to one or more of claims 1 to 5, **characterized in that**
R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R¹ represents a hydrogen atom; a linear or branched, saturated, optionally at least mono-substituted aliphatic group; an optionally at least mono-substituted aryl group;
most preferably R¹ represents a linear or branched, saturated, optionally at least mono-substituted C₁₋₆-alkyl group.

7. Compounds according to one or more of claims 1 to 6, **characterized in that**
R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system;
more preferably R¹⁹ represents a hydrogen atom; a linear or branched, saturated, optionally at least mono-substituted aliphatic group; an unbranched or branched, saturated or unsaturated, optionally at least mono-substituted alkoxy radical; an optionally at least mono-substituted aryl group;
most preferably R¹⁹ represents a linear or branched, saturated, optionally at least mono-substituted C₁₋₄-alkyl group; an optionally at least mono-substituted phenyl group.

8. Compounds according to claim 4, according to general formula (laa), 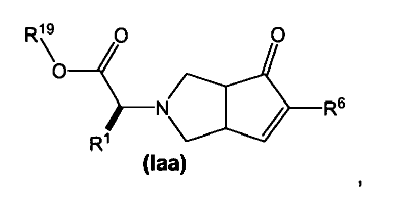 wherein
R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R⁶ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R⁶ represents a hydrogen atom; a linear or branched, saturated, optionally at least mono-substituted aliphatic group; an optionally at least mono-substituted aryl group;
most preferably R⁶ represents a linear or branched, saturated, optionally at least mono-substituted C₁₋₆-alkyl group; an optionally at least mono-substituted phenyl group.
and/or
R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹ represents a hydrogen atom; a linear or branched, saturated or unsaturated, optionally at least mono-substituted aliphatic group; an optionally at least mono-substituted aryl group, which may be condensed with an optionally at least mono-substituted mono- or polycyclic ring system;
more preferably R¹ represents a hydrogen atom; a linear or branched, saturated, optionally at least mono-substituted aliphatic group; an optionally at least mono-substituted aryl group;
most preferably R¹ represents a linear or branched, saturated, optionally at least mono-substituted C₁₋₆-alkyl group.
and/or
R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system; a branched or unbranched, saturated or unsaturated, optionally at least mono-substituted alkyl-aryl group in which the aryl group may be condensed with another, optionally at least mono-substituted mono- or polycyclic ring system; a saturated or unsaturated, optionally at least mono-substituted, optionally at least one heteroatom as ring member containing heterocyclyl group, which may be condensed with an optionally at least mono-substituted mono- or polycyclic ring system; or a branched or unbranched, saturated or unsaturated, optionally at least mono-substituted alkyl-heterocyclyl group in which the heterocyclyl group may be condensed with another, optionally at least mono-substituted mono- or polycyclic ring system;
preferably R¹⁹ represents a hydrogen atom; a linear or branched, saturated or unsaturated, optionally at least mono-substituted aliphatic group; an unbranched or branched, saturated or unsaturated, optionally at least mono-substituted alkoxy radical; an optionally at least mono-substituted aryl group, which may be condensed with an optionally at least mono-substituted mono-or polycyclic ring system;
more preferably R¹⁹ represents a hydrogen atom; a linear or branched, saturated, optionally at least mono-substituted aliphatic group; an unbranched or branched, saturated or unsaturated, optionally at least mono-substituted alkoxy radical; an optionally at least mono-substituted aryl group;
most preferably R¹⁹ represents a linear or branched, saturated, optionally at least mono-substituted C₁₋₄-alkyl group; an optionally at least mono-substituted phenyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

9. Compounds according to one or more of claims 1 to 8, selected from the group consisting of:
[9] (R)-ethyl 2-((3aR,6aS)-4-oxo-5-phenyl-1,3a,4,6a-tetrahydrocyclopenta[c]pyrrol-2(1H)-yl)propanoate
[10] (R)-ethyl 2-((3aS,6aR)-4-oxo-5-phenyl-1,3a,4,6a-tetrahydrocyclopenta[c]pyrrol-2(1H)-yl)propanoate,
[11] (S)-methyl 2-((3aR,6aS)-4-oxo-5-phenyl-1,3a,4,6a-tetrahydrocyclopenta[c]pyrrol-2(1H)-yl)-2-phenylacetate,
[12] (S)-methyl 2-((3aS,6aR)-4-oxo-5-phenyl-1,3a,4,6a-tetrahydrocyclopenta[c]pyrrol-2(1H)-yl)-2-phenylacetate,
[13] (S)-methyl 2-((3aR,6aS)-5-butyl-4-oxo-1,3a,4,6a-tetrahydrocyclopenta[c]pyrrol-2(1H)-yl)-2-phenylacetate,
[14] (S)-methyl 2-((3aS,6aR)-5-butyl-4-oxo-1 ,3a,4,6a-tetrahydrocyclopenta[c]pyrrol-2(1H)-yl)-2-phenylacetate,
[15] (R)-ethyl 2-((3aR,6aS)-5-butyl-4-oxo-1,3a,4,6a-tetrahydrocyclopenta[c]pyrrol-2(1H)-yl)propanoate,
[16] (R)-ethyl 2-((3aS,6aR)-5-butyl-4-oxo-1,3a,4,6a-tetrahydrocyclopenta[c]pyrrol-2(1H)-yl)propanoate,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

10. A process for the preparation of substituted bicyclic tetrahydropyrrole compounds of general formula (I) by reacting one substituted pyrroline compound of general formula (II), 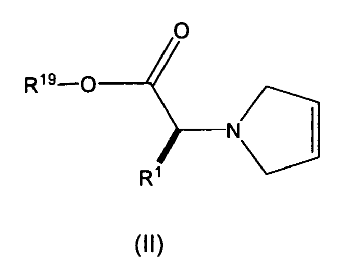 wherein
R¹ and R¹⁹ have the meaning according to claim 1, is reacted with a compound of general formula (III), 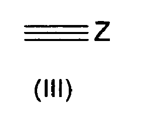 wherein Z represents a CH-R⁶ group; a CH-CHR⁷R^{7a} group; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group, to give compounds of general formula (la), 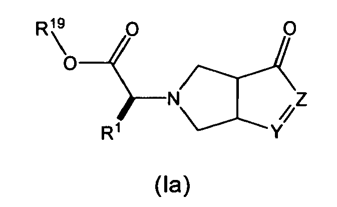 wherein the bond between Y and Z is unsaturated (Y=Z) in which Y represents a CH group and Z has the meaning according to claim 1.

11. A process for the preparation of substituted bicyclic tetrahydropyrrole compounds of general formula (I) by reacting one substituted pyrroline compound of general formula (Ia), 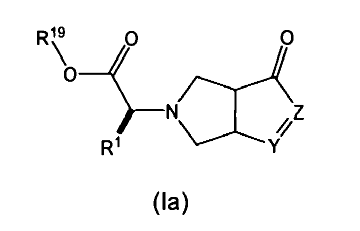 wherein R¹ and R¹⁹ have the meaning according to claim 1, Z represents a CH-R⁶ group; a CH-CHR⁷R^{7a} group; a CH-(C=O)-R⁸ group; a CH-CH₂(SO₂)-R⁹ group; a CH-CH₂(SO₂)-NR¹⁰R^{10a} group; or a CH-(C=O)-NR¹⁰R^{10a} group and Y represents a CH group, to give a compound of general formula (lb), 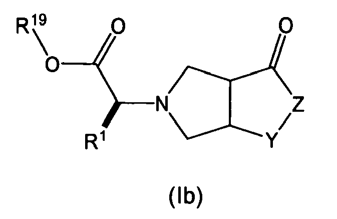 wherein R¹ and R¹⁹ have the meaning according to claim 1, Y and Z form a saturated (Y-Z) bond, and Y represents a CH₂ group; a C-R¹¹R¹² group; a CH-(C=O)-R ¹⁶ group; a CH-(SO₂)-R¹⁷ group; CH-(SO₂)-NR ¹⁸R^{18a} group; or a CH-(C=O)-NR¹⁸R^{18a} group.

12. A medicament comprising at least one substituted bicyclic tetrahydropyrrole compound of general formula (I), according to one or more of claims 1 to 9, said compound being optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof; or a prodrug thereof.

13. Use of a compound of general formula (l) according to one or more of claims 1 to 9 in the manufacture of a medicament.

14. Use of a compound of general formula (I) according to one or more of claims 1 to 9 in the manufacture of a medicament for the treatment or prophylaxis of a sigma receptor-mediated disease or condition.

15. Use according to claim 14, **characterized in that** the sigma receptor-mediated disease or condition is Alzheimer's disease.

16. Use according to claim 14, **characterized in that** the sigma receptor-mediated disease or condition is diarrhoea, lipoprotein disorders, migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety, psychosis or schizophrenia; inflammation, or autoimmune diseases.

17. Use according to claim 14, **characterized in that** the sigma receptor-mediated disease or condition is a disorder selected from the group consisting of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

18. Use according to claim 14, **characterized in that** the sigma receptor-mediated disease or condition is a disorder selected from the group consisting of pain, preferably neuropathic pain, inflammatory pain or other pain conditions involving allodynia and/or hyperalgesia.

19. Use of a compound of general formula (I) according to one or more of claims 1 to 9 as a pharmacological tool.
